# EUROPEAN PATENT APPLICATION

(11) **EP 3 808 842 A1**
(43) Date of publication of application: **21.04.2021**
(21) Application number: 19820313.5
(22) Date of filing: 07.06.2019
(51) Int. Cl.: C12N 9/78, A61K 38/50, A61K 47/64, A61P 37/02

(54) **PHARMACEUTICAL COMPOSITION FOR TREATING SEVERE COMPLEX IMMUNODEFICIENCY COMPRISING FUSION PROTEIN OF CELL PENETRATING PEPTIDE AND ADENOSINE DEAMINASE**

(30) Priority: 14.06.2018 KR 20180068375
(71) Applicant: Avixgen Inc., Seoul 06591 (KR)
(72) Inventor: JI, Eun Hye, Seoul 04990 (KR); AHN, Geon Young, Seoul 06211 (KR); BAEK, Yi Yong, Goyang-Si Gyeonggi-do 10375 (KR); CHOI, Jun Sub, Yongin-Si Gyeonggi-do 17027 (KR); KOO, Hye Cheong, Gwangmyeong-Si Gyeonggi-do 14314 (KR)
(74) Representative: Nony
(86) International application number: PCT/KR2019/006847
(87) International publication number: WO 2019/240429

(57) **Abstract**

The present disclosure relates to a composition including a conjugate of a cell-penetrating peptide and adenosine deaminase as an active ingredient. The composition may be useful for the treatment of severe combined immunodeficiency because the adenosine deaminase has increased cell permeability.

## Description

### Technical Field

The present disclosure relates to a conjugate of a cell-penetrating peptide and adenosine deaminase, and a pharmaceutical composition for treating severe combined immunodeficiency including the same.

### Background Art

Severe combined immunodeficiency (SCID) is a primary immunodeficiency disease characterized by deficient T lymphocytes and impaired B lymphocyte-mediated immune function due to abnormalities in genes involved in the development of the immune system. Patients with severe combined immunodeficiency are very vulnerable to infections with bacteria, viruses, or fungi, because there are little or no T lymphocytes and B lymphocytes involved in the immune systems of the patients. In addition, microorganisms that do not cause severe infections in healthy people may cause life-threatening infections in patients with severe combined immunodeficiency. Even microorganisms that do not cause disease may cause severe infections in patients with severe combined immunodeficiency.

Severe combined immunodeficiency can be classified into X-linked severe combined immunodeficiency (X-SCID) and autosomal recessive severe combined immunodeficiency. Known examples of autosomal recessive combined immunodeficiency include adenosine deaminase (ADA) deficiency, Jak3 (Janus kinase 3) deficiency, IL-7Rα (interleukin-7 receptor α) deficiency, RAG1 (recombination activating gene 1), RAG2 deficiency, Artemis deficiency, and CD45 deficiency.

ADA deficiency is caused by mutation in a gene in the long arm (20q12-13.11) of chromosome 20, which encodes the enzyme adenosine deaminase (ADA). ADA is an enzyme essential for the metabolism of T lymphocytes, and if the enzyme is deficient, toxic metabolites accumulate in lymphocytes, resulting in lymphocyte death. Methods of treating ADA include a method of transplanting hematopoietic stem cells having a normal ADA gene, and an enzyme replacement therapy method of administering PEG-ADA (polyethylene glycol-modified adenosine deaminase; Andagen) by intramuscular injection.

Meanwhile, cell-penetrating peptides (CPPs) are cell membrane-permeable peptides, each consisting of a short peptide of about 10 to 60 amino acids, and are mostly derived from protein-transduction domains or membrane-translocating sequences. It is known that, unlike general intracellular transduction pathways of foreign substances, CPPs can move into cells without damaging the cell membrane, and can intracellularly deliver even DNAs or proteins known to not pass through the cell membrane.

The present inventors have conducted studies on a cell-penetrating peptide derived from the HIV (human immunodeficiency virus) nucleocapsid, and have found that the cell-penetrating peptide can increase the cell permeability of ADA, thereby completing the present disclosure.

### DISCLOSURE

### Technical Problem

It is an object of the present disclosure to provide a conjugate including a cell-penetrating peptide and adenosine deaminase, a pharmaceutical composition for treating severe combined immunodeficiency including the same as an active ingredient, a method for treating severe combined immunodeficiency, and the use thereof.

### Technical Solution

One aspect of the present disclosure provides a conjugate including: a cell-penetrating peptide including the amino acid sequence of SEQ ID NO: 1; and adenosine deaminase set forth in the amino acid sequence of SEQ ID NO: 2.

Another aspect of the present disclosure provides a polynucleotide encoding the conjugate.

Still another aspect of the present disclosure provides a recombinant vector including the polynucleotide.

Yet another aspect of the present disclosure provides a host cell including the recombinant vector.

Still yet another aspect of the present disclosure provides a method for producing a conjugate, the method including steps of: (a) culturing the host cell in a culture medium; and (b) recovering the conjugate from the culture medium.

A further aspect of the present disclosure provides a pharmaceutical composition for treating severe combined immunodeficiency including the conjugate as an active ingredient.

According to one embodiment of the present disclosure, the severe combined immunodeficiency may be adenosine deaminase deficiency.

Another further aspect of the present disclosure provides a method for treating severe combined immunodeficiency, the method including a step of administering the composition to a subject.

Still another further aspect of the present disclosure provides the use of the conjugate in the manufacture of a medicament for treating severe combined immunodeficiency.

### Advantageous Effects

The conjugate of the cell-penetrating peptide and adenosine deaminase may be useful for the treatment of severe combined immunodeficiency, because the adenosine deaminase has increased cell permeability.

### Brief Description of Drawings

FIG. 1A illustrates the schematic structure of an advanced cell penetrating peptide-adenosine deaminase (ACP-ADA or ADA-ACP) protein, and FIG. 1B shows the results of analyzing whether a recombinant protein was expressed in 293FT cells.
FIG. 2A shows the results of observing a fluorescence signal after co-transfection of an ACP-ADA or ADA-ACP expression vector with a pDsRed vector, and FIG. 2B shows the results of measuring the activities of ACP-ADA and ADA-ACP.
FIG. 3A shows the results of the purified ACP-ADA after expression in *E. coli,* and FIG. 3B shows the results of measuring the activity of the purified ACP-ADA.
FIG. 4A shows the results of observing fluorescence signals after treating HeLa cells with the purified ACP-ADA, and FIG. 4B is a graph showing the results of quantifying the fluorescence signals.
FIG. 5 shows the results of delivering ADA into ADA-deficient cells and identifying ADA in the cells. FIG. 5A shows the results of observing the ACP-ADA protein that penetrated the cells, and FIG. 5B shows the results of measuring the activity of ADA.
FIG. 6 shows the results obtained by injecting each of bovine ADA and ACP-ADA into ADA gene-expressing hetero (+/-) mice and measuring the ADA activity in mononuclear cells isolated from the blood.

### Best Mode

To achieve the above objects, one aspect of the present disclosure provides a conjugate including: a cell-penetrating peptide including the amino acid sequence of SEQ ID NO: 1; and adenosine deaminase set forth in the amino acid sequence of SEQ ID NO: 2.

As used herein, the term "cell-penetrating peptide (CPP)" refers to a cell membrane-permeable peptide consisting of a short peptide of about 10 to about 60 amino acids, which can move into a cell without damaging the cell membrane and can intracellularly deliver even DNAs or proteins that cannot pass through the cell membrane.

As used herein, the term "adenosine deaminase" is an enzyme that deaminates the amino group of adenosine and catalyzes a process in which adenosine is hydrolyzed to produce inosine and ammonia (NH₃).

As used herein, the term "conjugate" refers to a substance in which a cell-penetrating peptide and a biologically or pharmaceutically active protein are linked together by a chemical/physical covalent or non-covalent bond.

In one embodiment of the present disclosure, the expression "biologically or pharmaceutically active protein" capable of forming the conjugate by binding to the cell-penetrating peptide means a protein that can regulate physiological phenomena *in vivo.* The expression includes proteins, peptides, lipids linked to proteins or peptides, carbohydrates, chemical compounds, or fluorescent labels. For example, adenosine deaminase having the amino acid sequence of SEQ ID NO: 2 may be used as the protein. The cell-penetrating peptide may be linked to the C-terminus or N-terminus of adenosine deaminase to form the conjugate.

In one embodiment of the present disclosure, the cell-penetrating peptide including the amino acid sequence of SEQ ID NO: 1 may be encoded by the polynucleotide sequence of SEQ ID NO: 5, and the adenosine deaminase set forth in the amino acid sequence of SEQ ID NO: 2 may be encoded by the polynucleotide sequence of SEQ ID NO: 6.

In one embodiment of the present disclosure, the conjugate may include the amino acid sequence of SEQ ID NO: 3 (ACP-ADA) or SEQ ID NO: 4 (ADA-ACP).

Another aspect of the present disclosure provides a polynucleotide encoding the conjugate.

As used herein, the term "polynucleotide" refers to a polymer of deoxyribonucleotide or ribonucleotide that exists in a single-stranded or double-stranded form. The term includes RNA genome sequences, DNAs (gDNA and cDNA), and RNA sequences transcribed therefrom, and includes analogues of natural polynucleotide, unless otherwise indicated.

In one embodiment of the present disclosure, the polynucleotide includes not only a nucleotide sequence encoding the conjugate, but also a sequence complementary thereto. The complementary sequences include not only completely complementary sequences, but also substantially complementary sequences. In addition, the polynucleotide sequence may be modified, and such modifications include addition, deletion or non-conservative substitution or conservative substitution of nucleotides.

In one embodiment of the present disclosure, the polynucleotide encoding the conjugate may include the polynucleotide sequence set forth in SEQ ID NO: 7 (ACP-ADA) or SEQ ID NO: 8 (ADA-ACP).

Still another aspect of the present disclosure provides a recombinant vector including the polynucleotide encoding the conjugate.

As used herein, the term "vector" refers to a means for expressing a target gene in a host cell. Examples of the vector include, but are not limited to, plasmid vectors, cosmid vectors, and viral vectors such as bacteriophage vectors, adenoviral vectors, retroviral vectors and adeno-associated viral vectors. Vectors that may be used as the recombinant vector may be constructed by engineering plasmids (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19, etc.), phages (e.g., λgt4λB, λ-Charon, λΔz1, and M13, etc.), or viruses (e.g., CMV, SV40, etc.), which are generally in the art.

The recombinant vector may include the polynucleotide sequence and a promoter operatively linked to the polynucleotide sequence.

As used herein, the term "operatively linked" refers to a functional linkage between a nucleotide expression control sequence (e.g., a promoter sequence) and another nucleotide sequence, whereby the control sequence controls the transcription and/or translation of the other nucleotide sequence.

Recombinant vectors that may be used in the present disclosure may be constructed by engineering plasmids (e.g., pSC101, ColE1, pBR322, pUC8/9, pHC79, pUC19, pET, etc.), phages (e.g., λgt4λB, λ-Charon, λΔz1 and M13, etc.), or viruses (e.g., SV40, etc.), which are generally used in the art.

The recombinant vector may include a tag sequence facilitating purification of the conjugate of the cell-penetrating peptide and adenosine deaminase, for example, a continuous histidine codon, a maltose-binding protein codon, a Myc codon, etc. and may further include a fusion partner for increasing the solubility of the conjugate. In addition, the recombinant vector may include a sequence that is specifically cleaved by an enzyme in order to remove an unnecessary portion when the conjugate is expressed, an expression control sequence, and a marker or reporter gene sequence for identifying intracellular delivery.

Yet another aspect of the present disclosure provides a host cell including the recombinant vector, that is, a cell transformed with the recombinant vector.

A host cell that is capable of stably and consecutively cloning or expressing the recombinant vector may be any host cell publicly-known in the art. Prokaryotic cells include, for example, *E. coli* JM109, *E*. *coli* BL21, *E. coli* RR1, *E. coli* LE392, *E. coli* B, *E. coli* X 1776, and *E. coli* W3110. When the recombinant vector is transformed into eukaryotic cells, *Saccharomyce cerevisiae,* insect cells, plant cells and animal cells, for example, SP2/0, CHO (Chinese hamster ovary) K1, CHO DG44, PER.C6, W138, BHK, COS-7, 293, HepG2, Huh7, 3T3, RIN and MDCK cell lines, may be used as host cells.

The polynucleotide or the recombinant vector containing the same may be transferred into the host cell using a transfer method widely known in the art. As the transfer method, for example, when the host cell is a prokaryotic cell, a CaCl₂ method or an electroporation method may be used, and when the host cell is a eukaryotic cell, a microinjection method, a calcium phosphate precipitation method, an electroporation method, a liposome-mediated transfection method or a gene bombardment method may be used, but the transfer method is not limited thereto.

A method of selecting the transformed host cell may be easily carried out using a phenotype expressed by a selection marker according to a method known in the art. For example, when the selection marker is a specific antibiotic resistance gene, the transformant may be easily selected by culturing the transformant in a medium containing the antibiotic.

Still yet another aspect of the present disclosure provides a method for producing a conjugate of a cell-penetrating peptide and adenosine deaminase, the method including steps of: culturing the host cell in a culture medium; and recovering the conjugate from the culture medium.

In one embodiment of the present disclosure, the culture of the cell may be large-scale cell culture, and the culture of the cell may be performed using a cell culture method which is commonly used. For example, the cell culture method may be any one or more selected from the group consisting of batch culture, repeated batch culture, fed-batch culture, repeated fed-batch culture, continuous culture, and perfusion culture, but is not limited thereto.

In one embodiment of the present disclosure, the step of recovering the conjugate from the culture medium may be performed using various separation and purification methods publicly-known in the art. Generally, the cell lysate may be centrifuged to remove cell debris, culture impurities, etc., and then precipitation, for example, salting out (ammonium sulfate precipitation and sodium phosphate precipitation), solvent precipitation (protein fraction precipitation using acetone, ethanol, isopropyl alcohol, etc.) or the like, may be performed, and dialysis, electrophoresis, and various column chromatographies may be performed.

A further aspect of the present disclosure provides a pharmaceutical composition for treating severe combined immunodeficiency including the conjugate as an active ingredient.

As used herein, the term "severe combined immunodeficiency (SCID)" refers to a disease characterized by deficient T lymphocytes and impaired B lymphocyte-mediated immune function due to gene abnormalities. Patients with this disease suffer from chronic infections because the immune system thereof does not function normally.

In one embodiment of the present disclosure, the severe combined immunodeficiency may be adenosine deaminase deficiency. If adenosine deaminase is deficient, toxic metabolites accumulate in lymphocytes, resulting in lymphocyte death, and hence T lymphocytes and B lymphocytes cannot develop normally.

The pharmaceutical composition of the present disclosure may include a pharmaceutically acceptable carrier, if necessary, in addition to the conjugate.

These pharmaceutically acceptable carriers are those that are commonly used in the manufacture of pharmaceuticals, and examples thereof include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like. In addition, the pharmaceutical composition of the present disclosure may further include additives such as a lubricant, a wetting agent, a sweetener, a flavoring agent, an emulsifying agent, a suspending agent, a preservative, etc.

The carrier may be included in an amount of about 1 wt% to about 99.99 wt%, preferably about 90 wt% to about 99.99 wt%, based on the total weight of the pharmaceutical composition of the present disclosure, and the additives may be included in an amount of about 0.1 wt% to about 20 wt%, based on the total weight of the pharmaceutical composition of the present disclosure.

Meanwhile, the pharmaceutical composition of the present disclosure may be administered orally or parenterally, but may be administered directly to the skin by a topical administration method.

The pharmaceutical composition of the present disclosure may be formulated with a pharmaceutically acceptable carrier and/or excipient and prepared in a unit dose form or contained in a multi-dose container. In this regard, the formulation may be in the form of a solution, a suspension or an emulsion, or may include elixir, extract, powder, granule, tablet, plaster, liniment, lotions, ointment, etc.

The daily dose of the pharmaceutical composition of the present disclosure may generally be in the range of 0.001 to 150 mg/kg of body weight, and may be administered once or several times. However, the dose of the pharmaceutical composition of the present disclosure is determined in view of various related factors such as the route of administration, the patient's age, sex and body weight, and the severity of the patient, and thus the dose should not be understood to limit the scope of the present disclosure in any way.

Another further aspect of the present disclosure provides a method for treating severe combined immunodeficiency, the method including a step of administering the composition to a subject.

The subject refers to an animal, and may typically be a mammal capable of exhibiting a beneficial effect by treatment with the conjugate of the present disclosure. Preferred examples of such subjects may include primates such as humans. In addition, such subjects may include all subjects who have symptoms of severe combined immunodeficiency or are at risk of having such symptoms.

Still another further aspect of the present disclosure provides the use of the conjugate in the manufacture of a medicament for treating severe combined immunodeficiency.

### Mode for Invention

Hereinafter, the present disclosure will be described in more detail with reference to one or more examples. However, these examples are to illustrate the present disclosure, and the scope of the present disclosure is not limited to these examples.

### Experimental Methods

### 1. Construction of Recombinant Vector for Expressing Cell Penetrating Peptide-Adenosine Deaminase Conjugate

As a cell-penetrating peptide, ACP (advanced cell-penetrating peptide (hereinafter referred to as ACP; SEQ ID NO: 1) was used. To express a recombinant protein consisting of a conjugate of ACP and adenosine deaminase (hereinafter referred to as ADA; SEQ ID NO: 2), a recombinant vector was constructed.

### 1-1. Amplification of ADA Gene

Restriction enzyme recognition sequences were added so that HindIII (New England Biolabs, NEB; USA) could act on the N-terminus of human ADA gene and XhoI (New England Biolabs) could act on the C-terminus of the human ADA gene. To this end, polymerase chain reaction (hereinafter referred to as PCR) was performed using the genomic DNA of the 293FT cell line as a template and PCR primers including restriction enzyme recognition sequences. Tables 1 and 2 below show the primer sequences and PCR conditions used in the PCR.

**[Table 1]**

| | SEQ ID NOs. | Sequences |
|---|---|---|
| Forward from HindIII-ADA | SEQ ID NO: 9 | CCC AAG CTT GGC CCA GAC GCC CGC |
| Reverse from ADA-XhoI | SEQ ID NO: 10 | CCC TCG AGG AGG TTC TGC CCT GCA GAG |

**[Table 2]**

| PCR reactants | | PCR cycles | | |
|---|---|---|---|---|
| dH₂O | 35.5 µl | 94°C | 2 min | |
| 2.5 mM dNTP | 4 µl | 94°C | 30 sec | 30 cycles |
| F primer (10 µM) | 2 µl | 55°C | 30 sec | |
| R primer (10 µM) | 2 µl | 72°C | 30 sec | |
| Template | 1 µl | 72°C | 5 min | |
| Taq polymerase | 0.5 µl | 4°C | Infinity | |
| 10x buffer | 5 µl | | | |
| Total | 50 µl | | | |

### 1-2. Construction of Recombinant Vector

A pTriEx-1.1 vector containing an ACP sequence and the PCR product of the above section 1-1 were treated and cleaved with HindIII and XhoI at 37°C for 2 hours, and the pTriEx vector fragment and the PCR product were isolated using a PCR Purification Kit (Cosmo, Korea) according to the manufacturer's protocol.

The isolated pTriEx-1.1 vector fragment and PCR product were ligated together at 4°C for 24 hours. The ligation conditions are shown in Table 3 below.

**[Table 3]**

| | |
|---|---|
| dH₂O | 4.1 µl |
| T4 DNA ligase buffer (10x) | 1 µl |
| HindIII-ADA-XhoI PCR product (30 ng) | 1.6 µl |
| ACP-containing pTriEx-1.1 vector (50 ng) | 2.3 µl |
| T4 DNA ligase (400 units/µl) | 1 µl |
| Total | 10 µl |

Meanwhile, a recombinant vector, in which the ADA gene was linked to the N-terminus of ACP (ADA-ACP), was constructed in the same manner as described above, except that NcoI and HindIII were used instead of HindIII and XhoI.

### 1-3. Transformation and DNA Sequencing

Competent cells and the ligation product were mixed together, and then incubated at 42°C for 1 minute. Thereafter, the competent cells were cultured in LB liquid medium at 37°C for 1 hour, and the cell culture was plated on an antibiotic-containing LB solid medium and cultured at 37°C for 16 hours. After completion of culture, the *E*. *coli* colony transformed with the correctly ligated recombinant vector was inoculated into LB liquid medium and shake-cultured at 37°C for 16 hours. After completion of culture, the cell culture was centrifuged, and the *E. coli* pellet was collected. From the collected *E. coli* pellet, the pTriEx ACP-ADA or pTriEx ADA-ACP recombinant vector was isolated using a plasmid extraction miniprep kit (Intron, Korea) according to the manufacturer's protocol. The isolated recombinant vector was measured for its concentration by a UV method and sequenced by Cosmo Co. Ltd. (Korea).

### 1-4. Construction of ADA-ACP Recombinant Vector for Bacterial Expression

The constructed pTriEx ACP-ADA and pTriEx ADA-ACP vectors were cleaved with NcoI and XhoI, and a pET28a vector was cleaved with the same restriction enzymes. Thereafter, the recombinant vectors were isolated in the same manner as described in the above sections 1-2 and 1-3 and were sequenced.

### 2. Analysis of Expression of ACP-ADA or ADA-ACP in Animal Cells

### 2-1. Analysis of Expression of ACP-ADA or ADA-ACP

The 293FT cell line was cultured with DMEM supplemented with 10% FBS and 100 U/ml penicillin/streptomycin in a humidified incubator at 37°C under 5% CO₂. The cultured 293FT cells were seeded into a 6-well plate at a density of 5x10⁵ cells/well and cultured for 24 hours. After completion of culture, the cells were transfected with 1 µg of the pTriEx ACP-ADA or pTriEx ADA-ACP recombinant vector DNA using Lipofector EZ reagent (APTABIO, Korea) according to the manufacturer's protocol. Thereafter, the cells were further cultured for 48 hours.

The cultured cells were washed with PBS and separated from the plate by pipetting. The separated cells were lysed by adding an RIPA buffer (10 mM Tris-Cl (pH 8.0), 1 mM EDTA, 0.5 mM EGTA, 1% Triton X-100, 0.1% sodium deoxycholate, 0.1% SDS, 140 mM NaCl and 1 mM PMSF) thereto, and left to stand on ice for 30 minutes. Thereafter, the cells were centrifuged (at 14,000 rpm for 20 min) at 4°C to remove cell debris, and only the cell lysate supernatant was collected. The protein concentration of the collected cell lysate was measured with Quick Start™ Bradford 1x Dye Reagent (Bio-Rad, USA), and 20 µg of the protein was separated by 10% SDS-PAGE (sodium dodecyl sulfate-polyacrylamide gel electrophoresis). The separated protein was transferred to a PVDF (polyvinylidene difluoride) membrane, and then the PVDF membrane was blocked with 5% skim milk. The PVDF membrane was incubated with primary antibodies (ACP, His and Actin antibodies) at 4°C for 16 hours, washed, and then incubated with secondary antibodies at room temperature for 1 hour. Thereafter, the protein band was detected using ECL (Pierce™ ECL Western Blotting Substrate (Thermo Scientific™, USA)) according to the manufacturer's protocol. The detection was performed using Amersham™ Imager 600 (GE Healthcare Life Science, USA).

### 2-2. Measurement of Adenosine Deaminase Activity of ACP-ADA or ADA-ACP

According to the same method as described in 2-1 above, the 293FT cell line was transfected with 1 µg of the pTriEx ACP-ADA or pTriEx ADA-ACP recombinant vector DNA and cultured for 48 hours. At this time, co-transfection of a pDsRed vector was performed to confirm whether transfection of the recombinant vector was achieved. After culture, the cells were washed once with PBS, and 1 ml of PBS was added to the plate, and the cells were separated by pipetting and centrifuged at 6,000 rpm for 3 minutes. The supernatant was removed, the cell pellet was collected, and then the ADA activity in the collected cell pellet was measured using an Adenosine Deaminase Activity Assay Kit (BioVision, USA) according to the manufacturer's protocol.

### 3. Analysis of Expression of ACP-ADA or ADA-ACP in E. coli

### 3-1. E. coli Culture and Lysis

In order to examine whether the pET28a ACP-ADA vector constructed in 1-4 above is expressed, an experiment was performed as follows.

*E. coli* BL21 (DE3) (Thermo Fisher) was transformed with each of the pET28a ACP-ADA vector and the pET28a-ADA vector and plated on LB (Luria-Bertani) solid medium, followed by culture at 37°C for 16 hours. After 16 hours, the formed colony was inoculated into LB liquid medium and additionally pre-cultured at 37°C. After about 16 hours, 250 µl of the cell pre-culture that reached an OD value of 0.5 to 0.6 was inoculated into 250 ml of LB liquid medium and cultured at 37°C for 3 to 4 hours until the OD value of the cell culture reached 0.5 to 0.6. When the OD value of the cell culture reached 0.5 to 0.6, 0.5 mM IPTG (isopropyl β-D-thiogalactoside) was added to the cell culture which was then additionally cultured at 37°C for 5 hours. After 5 hours, the cell culture was centrifuged, and the E. colipellet was collected. The collected pellet was suspended in a lysis buffer (20 mM Tris buffer and HCl 8.0, 50 mM KCl, 20 mM imidazole, and 1 mM EDTA). Then, the *E*. *coli* cells were lysed by sonication at an amplitude of 30 to 35%. The *E. coli* cell lysate was centrifuged and the supernatant was collected. The collected supernatant was filtered through a 0.45 µm filter.

### 3-2. Purification of ACP-ADA

Protein purification was performed using an AKTA Fast Protein Liquid Chromatography (FPLC) system (GE Healthcare Life Science). The Histap column (5 ml) was equilibrated with a pre-equilibration buffer (20 mM Tris-HCl (pH 8.0), 300 mM NaCl, 30 mM imidazole) and loaded with the supernatant collected in 3-1 above. Thereafter, protein was eluted with an elution buffer (20 mM Tris-HCl (pH 8.0), 300 mM NaCl, and 500 mM imidazole). The eluted fractions were analyzed by SDS-PAGE, and then only the fraction with high purity was collected, concentrated with Amicon (Millipore, USA) and subjected to buffer exchange with a storage buffer (20 mM Tris, 20% glycerol, and 1 mM DTT (dithiothreitol)). The fractions with no high purity were also concentrated with Amicon, and then purified by an anion-exchange chromatography column (Q-column). At this time, the column was equilibrated with a pre-equilibration buffer (20 mM Tris-HCl, pH 8.0), and protein was eluted with an elution buffer (20 mM Tris-HCl (pH 8.0), and 1 M NaCl). Each of the eluted fractions was analyzed by SDS-PAGE, and the fractions with high purity were concentrated and subjected to buffer exchange in the same manner as described above.

### 3-3. Measurement of Adenosine Deaminase Activity of ACP-ADA

The concentrations of the purified ACP-ADA and ADA were measured using Quick Start™ Bradford 1x Dye Reagent (Bio-Rad, USA). Based on the measured concentrations, 1, 2, 5 and 10 ng of the ACP-ADA were diluted, and 0.1, 0.2, 0.5 and 1 ng of the ADA were diluted. Using each diluted protein as a substrate, ADA activity was measured using an Adenosine Deaminase Activity Assay Kit (BioVision, USA).

### 4. Analysis of Cell Membrane Permeability of ACP-ADA

HeLa cells and GM02606 cells were cultured with DMEM supplemented with 10% FBS and 100 U/ml penicillin/streptomycin and RPMI1640 supplemented with 15% FBS and 100 U/ml penicillin/streptomycin, respectively, in a humidified incubator at 37°C under 5% CO₂. The cultured HeLa cells and GM02606 cells were seeded into 12-well plates containing glass at densities of 1x10⁵ cells/well and 8x10⁵ cells/well, respectively, and cultured for 24 hours. Thereafter, the cells were treated with 3 µM of the ACP-ADA purified in 3 above, and were cultured for 24 hours or 3 hours. Then, the cells were washed three times with PBS. The washed cells were fixed with 3.7% formaldehyde for 20 minutes and treated with PBS containing 0.2% Triton X-100 to increase the cell membrane permeability. Next, the cells were blocked with 3% BSA for 1 hour and incubated with an in-hose produced ACP antibody at room temperature for 2 hours, followed by washing three times with PBS. After washing, the cells were treated and incubated with Alexa 488 secondary antibody at room temperature for 1 hour, followed by washing three times with PBS. The cells were incubated with tubulin antibody (Santa Cruz Biotechnology, USA) at room temperature for 2 hours and washed 3 times with PBS. Thereafter, the cells were treated and incubated with Cy3-conjugated secondary antibody (Jackson ImmunoResearch, USA) at room temperature for 1 hour, followed by washing three times with PBS. Next, the cells were stained with DAPI (4',6-diamidino-2-phenylindol) for 10 minutes. The glasses having the HeLa cells and GM02606 cells attached thereto were detached, placed on slide glass, and observed by confocal laser scanning microscopy (LSM 700, Zeiss, Germany).

### 5. Analysis of Cell Membrane Permeability of ACP-ADA in ADA-Deficient Cells

### 5-1. Analysis of Permeability of ACP-ADA

The GM02606 cell line was cultured with RPMI1640 supplemented with 15% FBS and 100 U/ml penicillin/streptomycin in a humidified incubator at 37°C under 5% CO₂. The cultured GM02606 cells were seeded into a 6-well plate at a density of 2x10⁶ cells/well and cultured for 24 hours. Thereafter, the cells were treated with 3 µM of the ACP-ADA purified in 3 above and were cultured for 3 hours. Then, the cells were harvested and centrifuged at 5,000 rpm for 5 minutes. The separated cells were lysed by adding an RIPA buffer (10 mM Tris-Cl (pH 8.0), 1 mM EDTA, 0.5 mM EGTA, 1% Triton X-100, 0.1% sodium deoxycholate, 0.1% SDS, 140 mM NaCl and 1 mM PMSF) thereto, and left to stand on ice for 30 minutes. Thereafter, the cells were centrifuged (at 14,000 rpm for 20 min) at 4°C to remove cell debris, and only the cell lysate supernatant was collected. The protein concentration of the isolated cell lysate was measured using Pierce™ BCA protein assay kit (Thermo, USA), and 20 µg of the protein was separated by 10% SDS-PAGE (sodium dodecyl sulfate-polyacrylamide gel electrophoresis). The separated protein was transferred to a PVDF (polyvinylidene difluoride) membrane, and then the PVDF membrane was blocked with 5% skim milk. The PVDF membrane was incubated with primary antibodies (ADA, ACP and actin antibodies) at 4°C for 16 hours, washed, and then incubated with secondary antibodies at room temperature for 3 hours. Thereafter, the protein band was detected using ECL (Pierce™ ECL Western Blotting Substrate (Thermo Scientific™, USA)) according to the manufacturer's protocol. For detection of the protein, Amersham™ Imager 600 (GE Healthcare Life Science, USA) was used.

### 5-2. Measurement of Adenosine Deaminase Activity of ACP-ADA

According to the same method as described in 5-1 above, the GM02606 cell line was treated with 3 mM of the ACP-ADA purified in 3 above and was cultured for 3 hours. After culture, the cells were harvested and centrifuged at 5,000 rpm for 5 minutes.

Next, the supernatant was removed, and then the cell pellet was collected. Thereafter, the ADA activity in the cell pellet was measured using an Adenosine Deaminase Activity Assay Kit (BioVision, USA) according to the manufacturer's protocol. Specifically, in order to measure uric acid produced by further reacting ionsine produced by ADA, cell and plasma samples were reacted with the reagent in the kit, and then the absorbance at a wavelength of 293 nm was measured using an ELISA reader.

### 6. Measurement of Adenosine Deaminase Activity of ACP-ADA in ADA Gene-Expressing Hetero (+/-) Mice

### 6-1. Preparation of Experimental Animals

**For** use as experimental animals, parental mice (JAX #003265 - FVB;129-Adatm1Mw Tg(PLADA)4118Rkmb/J, Michael R. Blackburn et al., 1998, Melissa B. Aldrich et al., 2003) were purchased from Jackson Laboratory and mated, and then ADA gene-expressing hetero mice were obtained and used. The animal breeding room was maintained at a temperature of 23±3°C and a humidity of 55±15%. In addition, the breeding room was maintained under 12-hr light/dark cycles. Animal experiments were approved by the Institutional Animal Case and Use Committee of Knotus Co., Ltd., and animals were treated according to the instructions in the protocol.

### 6-2. Administration of Protein to ADA Hetero (+/-) Mice

100 U/kg of purchased bovine ADA (Creative Enzyme, USA) and the purified ACP-ADA protein was administered intravenously. The blood from ADA gene-expressing hetero (+/-) mice to which no substance is administered was used as a control. 1, 6 and 24 hours after administration of the protein, the corresponding animals were anesthetized with ether, and about 0.7 ml of blood was collected from the posterior vena cava by a syringe. Then, 7 µl of heparin (500 IU/ml sodium heparin) was added to the blood sample.

### 6-3. Analysis of Adenosine Deaminase Activity of ACP-ADA

The blood isolated from the mice was centrifuged at 1,000 rpm for 10 minutes. After centrifugation, only the blood was collected, placed on Histopaque-1077 solution, and centrifuged at 400xg for 30 minutes to separate the same into red blood cells (RBCs), mononuclear cells and plasma. Among the separated cells, only the mononuclear cells were collected and 1X PBS was added thereto, followed by centrifugation at 250xg for 10 minutes. Thereafter, the supernatant was removed, and the cell pellet was collected. Then, the ADA activity in the cell pellet was measured using an Adenosine Deaminase Activity Assay Kit (BioVision, USA) according to the manufacturer's protocol.

### Experimental Results

### 1. Analysis of Expression of ACP-ADA and ADA-ACP Vectors

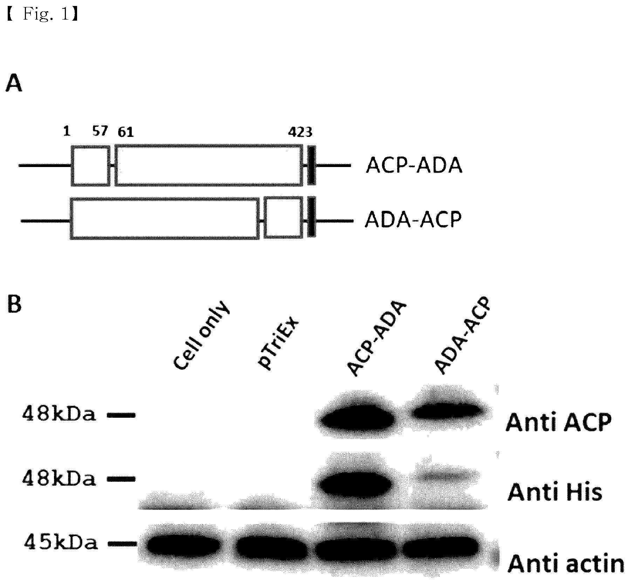

As shown in FIG. 1A, in order to examine whether the constructed ACP-ADA and ADA-ACP vectors are expressed in cells, 293FT cells were transfected with each of the vectors, and then analyzed by Western blot analysis. The dark gray square in FIG. 1A represents the His tag bound to the C-terminus of each of the recombinant proteins (ACP-ADA and ADA-ACP).

As a result, as shown in FIG. 1B, a band corresponding to the predicted size (48 kDa) of each of the recombinant proteins could be found in the Western blot analysis performed using the ACP antibody and the Hisspecific antibody bound to the C-terminus of each of the recombinant proteins (ACP-ADA and ADA-ACP). This result suggests that ACP-ADA and ADA-ACP were normally expressed in the cells.

### 2. Analysis of Adenosine Deaminase Activities of ACP-ADA and ADA-ACP

### 2-1. Analysis of Activities of ACP-ADA and ADA-ACP in Cells

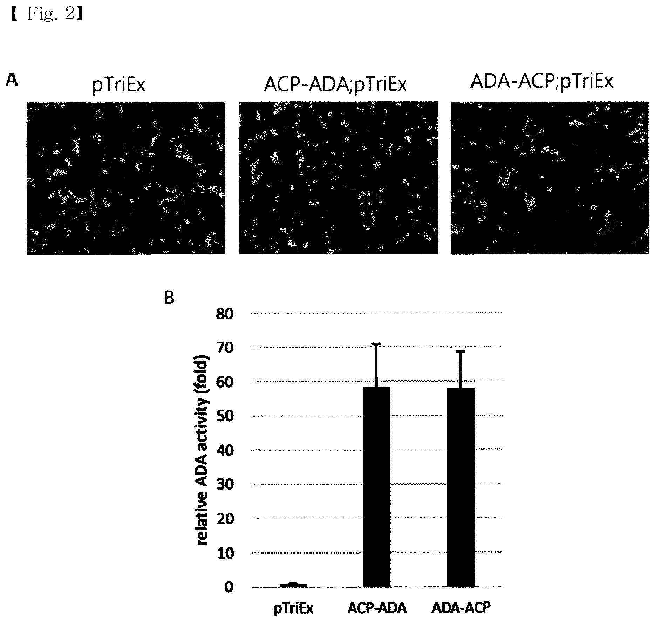

The activities of ACP-ADA and ADA-ACP in 293FT cells were measured, and as a result, as shown in FIG. 2B, it could be confirmed that, when the cells were transfected with ACP-ADA or ADA-ACP, the activity of ADA significantly increased by 50 times or more compared to that of the control group (pTriEx). On the other hand, as shown in FIG. 2A, it could be seen that the transfection efficiency did not significantly differ between the experimental groups.

Through this experiment, it could be confirmed that the ACP-ADA and ADA-ACP proteins expressed in the cells had ADA activity.

### 2-2. Analysis of Activity of Purified ACP-ADA

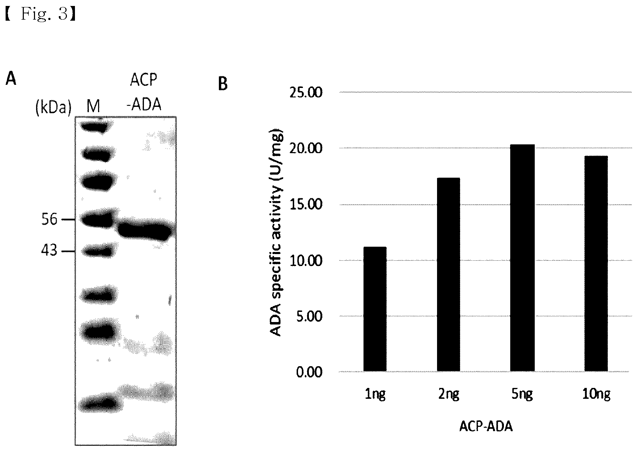

**The** activity of ACP-ADA isolated and purified from *E*. *coli* BL21(DE3) was measured, and as a result, as shown in FIG. 3B, it could be confirmed that, as the protein concentration increased, the ACP-ADA activity increased, and then converged to a constant value. Through this experiment, it could be seen that the specific activity of the purified ACP-ADA was 20 U/mg.

### 3. Analysis of Cell Membrane-Penetrating Activity of ACP-ADA

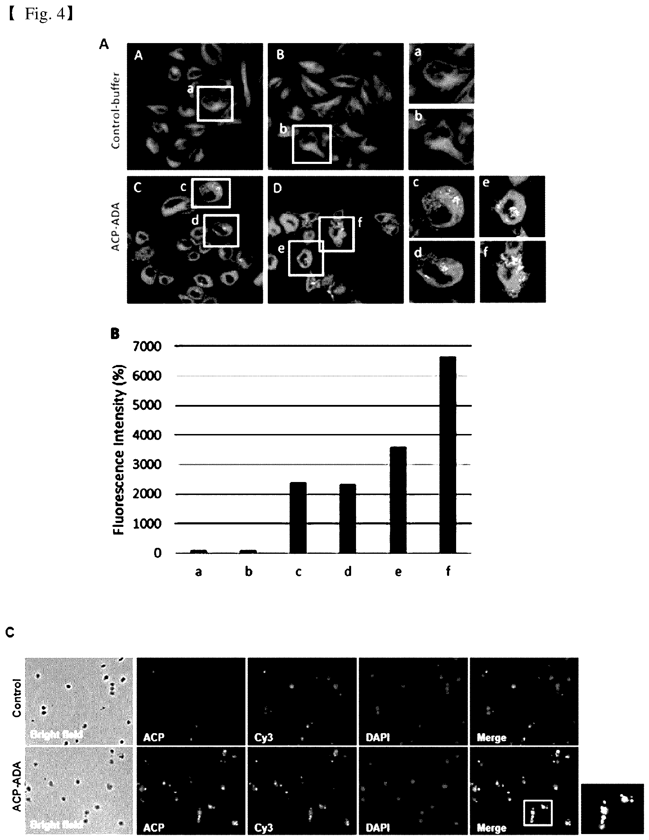

HeLa cells and GM02606 cells were treated with 3 µM of ACP-ADA for 24 hours and 3 hours, respectively, and then the cell permeability of ACP-ADA was analyzed. As a result, as shown in FIGS. 4A and 4C, it could be confirmed that, in the group treated with ACP-ADA, ACP-ADA entered the cells, and thus green fluorescence appeared (tubulin-red fluorescence, and nucleus-blue fluorescence). In addition, as shown in FIG. 4B, it could be confirmed that the intensity of the green fluorescence signal in the group treated with ACP-ADA was significantly higher than that in the control group.

### 4. Analysis of Penetration of ACP-ADA into ADA-Deficient Cells and Adenosine Deaminase Activity

In order to examine whether ACP-ADA penetrates ADA-deficient cells and has increased ADA activity, using the GM02606 cell line characterized by ADA deficiency, the intracellular penetration of ACP-ADA and the effect of ACP-ADA on increased ADA activity were analyzed.

### 4-1. Analysis of Penetration of ACP-ADA into Cells

In order to examine whether ACP-ADA purified from *E*. *coli* penetrates ADA-deficient cells, the ACP-ADA was allowed to penetrate the cell membrane of GM02606 cells, and then Western blot analysis was performed.

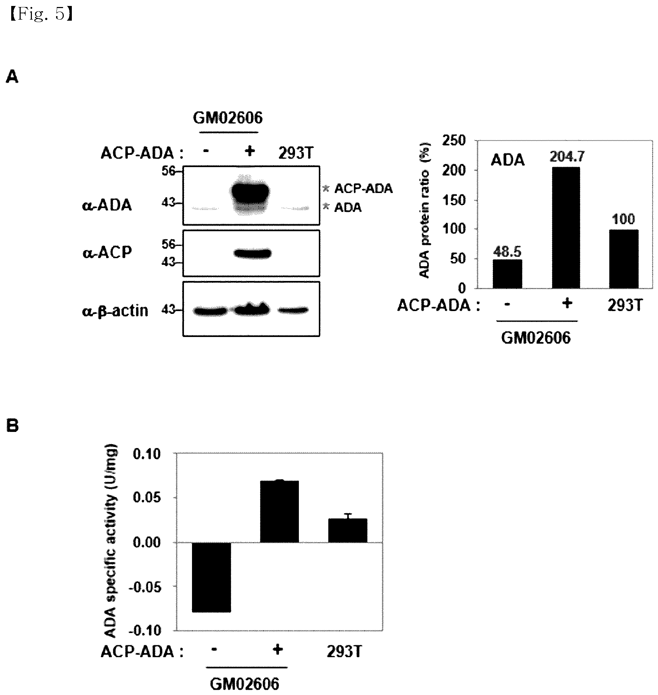

As a result, as shown in FIG. 5A, it could be confirmed that a band corresponding to the predicted size (48 kDa) of the recombinant protein was found in the Western blot analysis performed using ADA and ACP antibodies, suggesting that the ACP-ADA penetrated the cells.

### 4-2. Analysis of Activity of ACP-ADA in Cells

ACP-ADA purified from *E*. *coli* was allowed to penetrate the cell membrane of ADA-deficient cells (GM02606), and then the activity thereof was measured. As a result, as shown in FIG. 5B, it could be confirmed that the activity of ADA in the GM02606 cells, into which ACP-ADA was allowed to penetrate, significantly increased compared to that in the control group (GM02606).

Through this experiment, it could be confirmed that the ACP-ADA protein delivered into the cells had ADA activity.

### 5. Analysis of Deaminase Activity of ACP-ADA in ADA Hetero (+/-) Mice

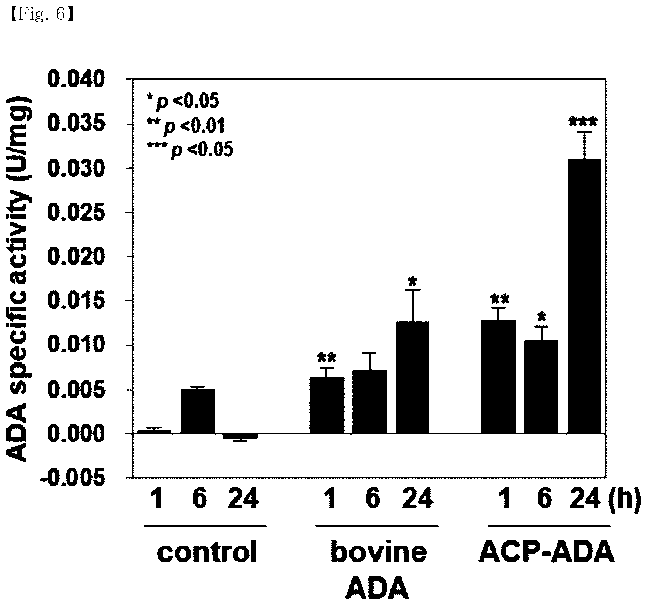

1, 6 and 24 hours after each of purchased bovine ADA and the ACP-ADA purified from *E. coli* was injected into ADA hetero (+/-) mice, the blood was collected, and the activities of ADA and ACP-ADA in the mononuclear cells were measured. As a result, as shown in FIG. 6, it could be confirmed that, 1 and 6 hours after injection of bovine ADA and 1, 6 and 24 hours after injection of ACP-ADA, the deaminase activity statistically significantly increased compared to that in the control group. In particular, it could be confirmed that the ADA activity in the mononuclear cells in the blood collected 24 hours after injection of ACP-ADA increased by about 3 times that in the control group.

Through this experiment, it could be confirmed that the ACP-ADA protein retained ADA activity even *in vivo.*

Those skilled in the art to which the present invention pertains will appreciate that the present disclosure may be embodied in modified forms without departing from the essential characteristics of the present disclosure. Therefore, it should be understood that the disclosed embodiments are illustrative in all aspects and are not restrictive. The scope of the present disclosure is defined by the claims rather than the foregoing description, and all differences within the scope equivalent to the claims should be construed as falling within the scope of the present invention.

## Claims

1. A conjugate comprising:
a cell-penetrating peptide comprising the amino acid sequence of SEQ ID NO: 1; and adenosine deaminase set forth in the amino acid sequence of SEQ ID NO: 2.

2. A polynucleotide encoding the conjugate of claim 1.

3. A recombinant vector comprising the polynucleotide of claim 2.

4. A host cell comprising the recombinant vector of claim 3.

5. A method for producing a conjugate, the method comprising steps of:
(a) culturing the host cell of claim 4 in a culture medium; and
(b) recovering the conjugate from the culture medium.

6. A pharmaceutical composition for treating severe combined immunodeficiency, the pharmaceutical composition comprising the conjugate of claim 1 as an active ingredient.

7. The pharmaceutical composition of claim 6, wherein the severe combined immunodeficiency is adenosine deaminase deficiency.

8. A method for treating severe combined immunodeficiency, the method comprising a step of administering the composition of claim 6 to a subject.

9. Use of the conjugate of claim 1 in the manufacture of a medicament for treating severe combined immunodeficiency.


<110>     Avixgen Incorporation

<120>     Pharmaceutical Composition for Treatment of Severe Combined
          Immunodeficiency comprising Cell Penetrating Peptide and
          Adenosine Deaminase Conjugate

<130>     PN180154

<160>     10

<170>     KoPatentIn 3.0

<210>     1
<211>     57
<212>     PRT
<213>     Artificial Sequence

<220>
<223>     Avixgen's advanced cell-penetrating peptide(ACP) amino acids
          sequence


<400>     1
Met Gly Gln Arg Gly Asn Phe Arg Asn Gln Arg Lys Thr Val Lys Cys
  1               5                  10                  15

Phe Asn Cys Gly Lys Glu Gly His Ile Ala Lys Asn Cys Arg Ala Pro
             20                  25                  30

Arg Lys Lys Gly Cys Trp Arg Cys Gly Arg Glu Gly His Gln Met Lys
         35                  40                  45

Asp Cys Thr Glu Arg Gln Ala Asn Leu
     50                  55


<210>     2
<211>     363
<212>     PRT
<213>     Homo sapiens

<400>     2

<210> 3
<211> 430
<212> PRT
<213> Artificial Sequence

<220>
<223> ACP-ADA amino acids sequence

<400> 3
Met Gly Gln Arg Gly Asn Phe Arg Asn Gln Arg Lys Thr Val Lys Cys
1               5                   10                  15

Phe Asn Cys Gly Lys Glu Gly His Ile Ala Lys Asn Cys Arg Ala Pro

20                  25                  30

Arg Lys Lys Gly Cys Trp Arg Cys Gly Arg Glu Gly His Gln Met Lys
        35                  40                  45

Asp Cys Thr Glu Arg Gln Ala Asn Leu Thr Ser Leu Ala Gln Thr Pro
    50                  55                  60

Ala Phe Asp Lys Pro Lys Val Glu Leu His Val His Leu Asp Gly Ser
65                  70                  75                  80

Ile Lys Pro Glu Thr Ile Leu Tyr Tyr Gly Arg Arg Arg Gly Ile Ala
                85                  90                  95

Leu Pro Ala Asn Thr Ala Glu Gly Leu Leu Asn Val Ile Gly Met Asp
            100                 105                 110

Lys Pro Leu Thr Leu Pro Asp Phe Leu Ala Lys Phe Asp Tyr Tyr Met
        115                 120                 125

Pro Ala Ile Ala Gly Cys Arg Glu Ala Ile Lys Arg Ile Ala Tyr Glu
    130                 135                 140

Phe Val Glu Met Lys Ala Lys Glu Gly Val Val Tyr Val Glu Val Arg
145                 150                 155                 160

Tyr Ser Pro His Leu Leu Ala Asn Ser Lys Val Glu Pro Ile Pro Trp
                165                 170                 175

Asn Gln Ala Glu Gly Asp Leu Thr Pro Asp Glu Val Val Ala Leu Val
            180                 185                 190

Gly Gln Gly Leu Gln Glu Gly Glu Arg Asp Phe Gly Val Lys Ala Arg
        195                 200                 205

Ser Ile Leu Cys Cys Met Arg His Gln Pro Asn Trp Ser Pro Lys Val
    210                 215                 220

Val Glu Leu Cys Lys Lys Tyr Gln Gln Gln Thr Val Val Ala Ile Asp
225                 230                 235                 240

Leu Ala Gly Asp Glu Thr Ile Pro Gly Ser Ser Leu Leu Pro Gly His
                245                 250                 255

Val Gln Ala Tyr Gln Glu Ala Val Lys Ser Gly Ile His Arg Thr Val
            260                 265                 270

His Ala Gly Glu Val Gly Ser Ala Glu Val Val Lys Glu Ala Val Asp
        275                 280                 285

Ile Leu Lys Thr Glu Arg Leu Gly His Gly Tyr His Thr Leu Glu Asp
    290                 295                 300

Gln Ala Leu Tyr Asn Arg Leu Arg Gln Glu Asn Met His Phe Glu Ile
305                 310                 315                 320

Cys Pro Trp Ser Ser Tyr Leu Thr Gly Ala Trp Lys Pro Asp Thr Glu
                325                 330                 335

His Ala Val Ile Arg Leu Lys Asn Asp Gln Ala Asn Tyr Ser Leu Asn
            340                 345                 350

Thr Asp Asp Pro Leu Ile Phe Lys Ser Thr Leu Asp Thr Asp Tyr Gln

355                 360                 365

Met Thr Lys Arg Asp Met Gly Phe Thr Glu Glu Glu Phe Lys Arg Leu
    370                 375                 380

Asn Ile Asn Ala Ala Lys Ser Ser Phe Leu Pro Glu Asp Glu Lys Arg
385                 390                 395                 400

Glu Leu Leu Asp Leu Leu Tyr Lys Ala Tyr Gly Met Pro Pro Ser Ala
                405                 410                 415

Ser Ala Gly Gln Asn Leu Leu Glu His His His His His His
            420                 425                 430

<210>    4
<211>    431
<212>    PRT
<213>    Artificial Sequence

<220>
<223>    ADA-ACP amino acids sequence

<400>    4
Met Gly Ala Gln Thr Pro Ala Phe Asp Lys Pro Lys Val Glu Leu His
1               5                   10                  15

Val His Leu Asp Gly Ser Ile Lys Pro Glu Thr Ile Leu Tyr Tyr Gly
            20                  25                  30

Arg Arg Arg Gly Ile Ala Leu Pro Ala Asn Thr Ala Glu Gly Leu Leu
        35                  40                  45

Asn Val Ile Gly Met Asp Lys Pro Leu Thr Leu Pro Asp Phe Leu Ala
    50                  55                  60

Lys Phe Asp Tyr Tyr Met Pro Ala Ile Ala Gly Cys Arg Glu Ala Ile
65                  70                  75                  80

Lys Arg Ile Ala Tyr Glu Phe Val Glu Met Lys Ala Lys Glu Gly Val
                85                  90                  95

Val Tyr Val Glu Val Arg Tyr Ser Pro His Leu Leu Ala Asn Ser Lys
            100                 105                 110

Val Glu Pro Ile Pro Trp Asn Gln Ala Glu Gly Asp Leu Thr Pro Asp
        115                 120                 125

Glu Val Val Ala Leu Val Gly Gln Gly Leu Gln Glu Gly Glu Arg Asp
    130                 135                 140

Phe Gly Val Lys Ala Arg Ser Ile Leu Cys Cys Met Arg His Gln Pro
145                 150                 155                 160

Asn Trp Ser Pro Lys Val Val Glu Leu Cys Lys Lys Tyr Gln Gln Gln
                165                 170                 175

Thr Val Val Ala Ile Asp Leu Ala Gly Asp Glu Thr Ile Pro Gly Ser
            180                 185                 190

Ser Leu Leu Pro Gly His Val Gln Ala Tyr Gln Glu Ala Val Lys Ser
        195                 200                 205

Gly Ile His Arg Thr Val His Ala Gly Glu Val Gly Ser Ala Glu Val
    210                 215                 220

Val Lys Glu Ala Val Asp Ile Leu Lys Thr Glu Arg Leu Gly His Gly
225                 230                 235                 240

Tyr His Thr Leu Glu Asp Gln Ala Leu Tyr Asn Arg Leu Arg Gln Glu
                245                 250                 255

Asn Met His Phe Glu Ile Cys Pro Trp Ser Ser Tyr Leu Thr Gly Ala
            260                 265                 270

Trp Lys Pro Asp Thr Glu His Ala Val Ile Arg Leu Lys Asn Asp Gln
        275                 280                 285

Ala Asn Tyr Ser Leu Asn Thr Asp Asp Pro Leu Ile Phe Lys Ser Thr
    290                 295                 300

Leu Asp Thr Asp Tyr Gln Met Thr Lys Arg Asp Met Gly Phe Thr Glu
305                 310                 315                 320

Glu Glu Phe Lys Arg Leu Asn Ile Asn Ala Ala Lys Ser Ser Phe Leu
                325                 330                 335

Pro Glu Asp Glu Lys Arg Glu Leu Leu Asp Leu Leu Tyr Lys Ala Tyr
            340                 345                 350

Gly Met Pro Pro Ser Ala Ser Ala Gly Gln Asn Leu Lys Leu Gln Arg
        355                 360                 365

Gly Asn Phe Arg Asn Gln Arg Lys Thr Val Lys Cys Phe Asn Cys Gly
    370                 375                 380

Lys Glu Gly His Ile Ala Lys Asn Cys Arg Ala Pro Arg Lys Lys Gly
385                 390                 395                 400

Cys Trp Arg Cys Gly Arg Glu Gly His Gln Met Lys Asp Cys Thr Glu
                405                 410                 415

Arg Gln Ala Asn Leu Leu Glu His His His His His His His His
            420                 425                 430

<210>    5
<211>    171
<212>    DNA
<213>    Artificial Sequence

<220>
<223>    ACP polynucleotides

<400>    5
atgggccagc ggggaaactt caggaaccag agaaaaactg tgaagtgctt caattgcgga        60

aaggagggcc acatcgctaa gaactgccgc gcccccagaa agaaaggctg ctggagatgc       120

ggcagagagg gccaccagat gaaggactgc acagagagac aggcaaactt a                171

<210>    6

<211>    1092
<212>    DNA
<213>    Homo sapiens

<400>    6
atggcccaga cgcccgcctt cgacaagccc aaagtagaac tgcatgtcca cctagacgga      60

tccatcaagc ctgaaaccat cttatactat ggcaggagga gagggatcgc cctcccagct     120

aacacagcag aggggctgct gaacgtcatt ggcatggaca agccgctcac ccttccagac     180

ttcctggcca agtttgacta ctacatgcct gctatcgcgg gctgccggga ggctatcaaa     240

aggatcgcct atgagtttgt agagatgaag gccaaagagg gcgtggtgta tgtggaggtg     300

cggtacagtc cgcacctgct ggccaactcc aaagtggagc caatcccctg gaaccaggct     360

gaaggggacc tcaccccaga cgaggtggtg gccctagtgg gccagggcct gcaggagggg     420

gagcgagact tcggggtcaa ggcccggtcc atcctgtgct gcatgcgcca ccagcccaac     480

tggtccccca aggtggtgga gctgtgtaag aagtaccagc agcagaccgt ggtagccatt     540

gacctggctg gagatgagac catcccagga agcagcctct tgcctggaca tgtccaggcc     600

taccaggagg ctgtgaagag cggcattcac cgtactgtcc acgccgggga ggtgggctcg     660

gccgaagtag taaaagaggc tgtggacata ctcaagacag agcggctggg acacggctac     720

cacaccctgg aagaccaggc cctttataac aggctgcggc aggaaaacat gcacttcgag     780

atctgcccct ggtccagcta cctcactggt gcctggaagc cggacacgga gcatgcagtc     840

attcggctca aaaatgacca ggctaactac tcgctcaaca cagatgaccc gctcatcttc     900

aagtccaccc tggacactga ttaccagatg accaaacggg acatgggctt tactgaagag     960

gagtttaaaa ggctgaacat caatgcggcc aaatctagtt tcctcccaga agatgaaaag    1020

agggagcttc tcgacctgct ctataaagcc tatgggatgc caccttcagc ctctgcaggg    1080

cagaacctct ga                                                        1092

<210>    7
<211>    1293
<212>    DNA
<213>    Artificial Sequence

<220>
<223>    ACP-ADA polynucleotides

<400>    7
atgggccagc ggggaaactt caggaaccag agaaaaactg tgaagtgctt caattgcgga      60

aaggagggcc acatcgctaa gaactgccgc gcccccagaa agaaaggctg ctggagatgc     120

ggcagagagg gccaccagat gaaggactgc acagagagac aggcaaactt aacaagcttg     180

gcccagacgc ccgccttcga caagcccaaa gtagaactgc atgtccacct agacggatcc     240

atcaagcctg aaaccatctt atactatggc aggaggagag ggatcgccct cccagctaac     300

acagcagagg ggctgctgaa cgtcattggc atggacaagc cgctcaccct tccagacttc 360

ctggccaagt ttgactacta catgcctgct atcgcgggct gccgggaggc tatcaaaagg 420

atcgcctatg agtttgtaga gatgaaggcc aaagagggcg tggtgtatgt ggaggtgcgg 480

tacagtccgc acctgctggc caactccaaa gtggagccaa tcccctggaa ccaggctgaa 540

ggggacctca ccccagacga ggtggtggcc ctagtgggcc agggcctgca ggagggggag 600

cgagacttcg gggtcaaggc ccggtccatc ctgtgctgca tgcgccacca gcccaactgg 660

tcccccaagg tggtggagct gtgtaagaag taccagcagc agaccgtggt agccattgac 720

ctggctggag atgagaccat cccaggaagc agcctcttgc ctggacatgt ccaggcctac 780

caggaggctg tgaagagcgg cattcaccgt actgtccacg ccggggaggt gggctcggcc 840

gaagtagtaa aagaggctgt ggacatactc aagacagagc ggctgggaca cggctaccac 900

accctggaag accaggccct ttataacagg ctgcggcagg aaaacatgca cttcgagatc 960

tgcccctggt ccagctacct cactggtgcc tggaagccgg acacggagca tgcagtcatt 1020

cggctcaaaa atgaccaggc taactactcg ctcaacacag atgacccgct catcttcaag 1080

tccaccctgg acactgatta ccagatgacc aaacgggaca tgggctttac tgaagaggag 1140

tttaaaaggc tgaacatcaa tgcggccaaa tctagtttcc tcccagaaga tgaaaagagg 1200

gagcttctcg acctgctcta taaagcctat gggatgccac cttcagcctc tgcagggcag 1260

aacctcctcg agcaccacca ccaccaccac tga 1293

<210> 8
<211> 1296
<212> DNA
<213> Artificial Sequence

<220>
<223> ADA-ACP polynucleotides

<400> 8
atgggcgccc agacgcccgc cttcgacaag cccaaagtag aactgcatgt ccacctagac 60

ggatccatca agcctgaaac catcttatac tatggcagga ggagagggat cgccctccca 120

gctaacacag cagaggggct gctgaacgtc attggcatgg acaagccgct cacccttcca 180

gacttcctgg ccaagtttga ctactacatg cctgctatcg cgggctgccg ggaggctatc 240

aaaaggatcg cctatgagtt tgtagagatg aaggccaaag agggcgtggt gtatgtggag 300

gtgcggtaca gtccgcacct gctggccaac tccaaagtgg agccaatccc ctggaaccag 360

gctgaagggg acctcacccc agacgaggtg gtggccctag tgggccaggg cctgcaggag 420

ggggagcgag acttcggggt caaggcccgg tccatcctgt gctgcatgcg ccaccagccc 480

aactggtccc ccaaggtggt ggagctgtgt aagaagtacc agcagcagac cgtggtagcc 540

attgacctgg ctggagatga gaccatccca ggaagcagcc tcttgcctgg acatgtccag 600

gcctaccagg aggctgtgaa gagcggcatt caccgtactg tccacgccgg ggaggtgggc 660

tcggccgaag tagtaaaaga ggctgtggac atactcaaga cagagcggct gggacacggc 720

taccacaccc tggaagacca ggccctttat aacaggctgc ggcaggaaaa catgcacttc 780

gagatctgcc cctggtccag ctacctcact ggtgcctgga agccggacac ggagcatgca 840

gtcattcggc tcaaaaatga ccaggctaac tactcgctca acacagatga cccgctcatc 900

ttcaagtcca ccctggacac tgattaccag atgaccaaac gggacatggg ctttactgaa 960

gaggagttta aaaggctgaa catcaatgcg gccaaatcta gtttcctccc agaagatgaa 1020

aagagggagc ttctcgacct gctctataaa gcctatggga tgccaccttc agcctctgca 1080

gggcagaacc tcaagcttca gcggggaaac ttcaggaacc agagaaaaac tgtgaagtgc 1140

ttcaattgcg gaaaggaggg ccacatcgct aagaactgcc gcgcccccag aaagaaaggc 1200

tgctggagat gcggcagaga gggccaccag atgaaggact gcacagagag acaggcaaac 1260

ttactcgagc accaccatca ccatcaccat cactaa 1296

<210> 9
<211> 24
<212> DNA
<213> Artificial Sequence

<220>
<223> Forward from HindIII-ADA

<400> 9
cccaagcttg gcccagacgc ccgc 24

<210> 10
<211> 27
<212> DNA
<213> Artificial Sequence

<220>
<223> Reverse from ADA-XhoI

<400> 10
ccctcgagga ggttctgccc tgcagag 27